(19) Europäisches Patentamt European Patent Office Office européen des brevets

(11) **EP 4 702 921 A1**

(12) **EUROPEAN PATENT APPLICATION**
published in accordance with Art. 153(4) EPC

(43) Date of publication:
04.03.2026 Bulletin 2026/10

(21) Application number: 24795510.7

(22) Date of filing: 01.02.2024

(51) International Patent Classification (IPC):
$A61B\ 5/1455^{(2006.01)}$     $A61B\ 5/145^{(2006.01)}$
$A61B\ 5/00^{(2006.01)}$     $G06F\ 17/18^{(2006.01)}$

(52) Cooperative Patent Classification (CPC):
A61B 5/00; A61B 5/145; A61B 5/1455; G06F 17/18

(86) International application number:
PCT/CN2024/075234

(87) International publication number:
WO 2024/222121 (31.10.2024 Gazette 2024/44)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority: 28.04.2023 CN 202310483892

(71) Applicant: **Innolight Technology (Suzhou) Ltd.
Suzhou, Jiangsu 215000 (CN)**

(72) Inventors:
• LIU, Yong
Suzhou, Jiangsu 215000 (CN)
• CHEN, Ziang
Suzhou, Jiangsu 215000 (CN)
• LI, Xianyao
Suzhou, Jiangsu 215000 (CN)
• SUN, Yuzhou
Suzhou, Jiangsu 215000 (CN)

(74) Representative: **Becker, Eberhard
Becker Kurig & Partner
Patentanwälte mbB
Bavariastraße 7
80336 München (DE)**

(54) **METHOD AND APPARATUS FOR ANALYZING BLOOD GLUCOSE CONCENTRATION, ELECTRONIC DEVICE, AND STORAGE MEDIUM**

(57) The present application relates to a method and apparatus for analyzing a blood glucose concentration, a device, a storage medium, and a computer program product. The method comprises: acquiring intensity sets corresponding to a plurality of photoplethysmography pulse wave sets for a target object in a target time period, the intensity set comprising an average alternating-current intensity and an average direct-current intensity; performing curve fitting on the plurality of intensity sets to obtain a fitting coefficient and a fitting bias; comparing the fitting bias with a bias range corresponding to the target object, and when it is determined that the fitting bias is not within the bias range, adjusting the fitting bias to obtain an adjusted fitting bias; and performing, on the basis of the adjusted fitting bias, curve fitting on the plurality of intensity sets to obtain an adjusted fitting coefficient, and taking the adjusted fitting coefficient as the blood glucose concentration of the target object in the target time period. By adopting the method, the accuracy of blood glucose concentration analysis can be improved.

Acquiring intensity sets corresponding to multiple photoplethysmography pulse wave sets for a target object in a target time period; the intensity sets including an average alternating-current intensity and an average direct-current intensity — 202

Performing curve fitting on the intensity sets to obtain a fitting coefficient and a fitting bias — 204

Comparing the fitting bias with a bias range corresponding to the target object, and when it is determined that the fitting bias is not within the bias range, adjusting the fitting bias to obtain an adjusted fitting bias — 206

Performing, on the basis of the adjusted fitting bias, curve fitting on the intensity sets to obtain an adjusted fitting coefficient, and taking the adjusted fitting coefficient as the blood glucose concentration of the target object in the target time period — 208

FIG. 2

## Description

This application claims the priority benefit to China patent application No. 202310483892.4, with an invention title, "METHOD AND APPARATUS FOR ANALYZING BLOOD GLUCOSE CONCENTRATION, ELECTRONIC DEVICE, AND STORAGE MEDIUM", filed on April 28, 2023, in the China National Intellectual Property Administration. The entirety of the above-mentioned patent application is hereby incorporated by reference herein and made a part of this specification.

## Technical Field

The present application relates to the field of non-invasive blood glucose concentration analysis, and particularly relates to a method and an apparatus for analyzing a blood glucose concentration, an electronic device, a storage medium, and a computer program product.

## Related Art

With the development of sensor technology, wearable smart device has emerged. The wearable smart device is configured to acquire physiological characteristic parameters of a wearer. Through analysis of the physiological characteristic parameters, continuous monitoring of the wearer's physical condition is achieved.

In conventional technology, the wearable smart device adopts a method for detecting near-infrared non-invasive blood glucose to achieve continuous monitoring of the wearer's blood glucose concentration. Since this method is easily interfered with by human tissue, the detected blood glucose concentration has relatively low accuracy.

## Summary of invention

Based on this, it is necessary to provide a method and an apparatus for analyzing a blood glucose concentration, an electronic device, a computer-readable storage medium, and a computer program product that may improve accuracy in response to the aforementioned technical problems.

In a first aspect, the present application provides a method for analyzing a blood glucose concentration. The method includes:

acquiring intensity sets respectively corresponding to multiple photoplethysmography pulse wave sets for a target object in a target time period; the intensity sets include average alternating-current intensity and average direct-current intensity;

performing curve fitting on the intensity sets to obtain a fitting coefficient and a fitting bias;

comparing the fitting bias with a bias range corresponding to the target object, and in a situation when it is determined that the fitting bias is not within the bias range, adjusting the fitting bias to obtain an adjusted fitting bias;

performing, on a basis of the adjusted fitting bias, the curve fitting on the intensity sets to obtain an adjusted fitting coefficient, and taking the adjusted fitting coefficient as the blood glucose concentration of the target object in the target time period.

In an embodiment, after performing, on the basis the adjusted fitting bias, the curve fitting on the intensity sets to obtain the adjusted fitting coefficient, further includes:

acquiring a correction parameter value of the target object; the correction parameter value corresponds to a correction parameter, and the correction parameter includes at least one of physiological characteristic parameters and environmental characteristic parameters;

acquiring multiple correction weights corresponding to the target object; the correction weights include a correction weight corresponding to the adjusted fitting coefficient, and the correction weights corresponding to each of the correction parameters;

taking the adjusted fitting coefficient as the blood glucose concentration of the target object in the target time period includes:

fusing the adjusted fitting coefficient and each of the correction parameter values with corresponding correction weights to obtain the blood glucose concentration of the target object in the target time period.

In an embodiment, acquiring the intensity sets corresponding to the photoplethysmography pulse wave sets for the target object in the target time period includes:

acquiring the photoplethysmography pulse wave sets for the target object in the target time period; each of the photoplethysmography pulse wave sets corresponds to one emission intensity, and each of the photoplethysmography pulse wave sets includes at least two photoplethysmography pulse waves;

for each of the photoplethysmography pulse waves, performing filtering processing on the photoplethysmography pulse wave to obtain a direct-current signal and an alternating-current signal, on a basis of a signal intensity of the direct-current signal, determining a direct-current intensity corresponding to the photoplethysmography pulse wave, and on a basis of the alternating-current signal, determining an alternating-current intensity corresponding to the photoplethysmography pulse wave;

for each of the photoplethysmography pulse wave sets, averaging direct-current intensities corresponding to the photoplethysmography pulse waves to obtain an average direct-current intensity, averaging alternating-current intensities corresponding to the photoplethysmography pulse waves to obtain an average alternating-current intensity, and on a basis of the average direct-current intensity and the average alternating-current intensity, obtaining an intensity set corresponding to the photoplethysmography pulse wave set.

In an embodiment, for each photoplethysmography pulse wave, before performing the filtering processing on the photoplethysmography pulse wave to obtain the direct-current signal and the alternating-current signal, further includes:

performing a quality evaluation on the photoplethysmography pulse wave to obtain a quality evaluation result of the photoplethysmography pulse wave; the quality evaluation result comprising evaluation values corresponds to each band composing the photoplethysmography pulse wave;

removing bands corresponding to the evaluation values less than an evaluation threshold from the photoplethysmography pulse wave to obtain an adjusted photoplethysmography pulse wave;

for each photoplethysmography pulse wave, performing the filtering processing on the photoplethysmography pulse wave to obtain a direct-current signal and an alternating-current signal including:

for each of the adjusted photoplethysmography pulse waves, performing filtering processing on the adjusted photoplethysmography pulse wave to obtain the direct-current signal and the alternating-current signal.

In an embodiment, on the basis of the alternating-current signal, determining the alternating-current intensity corresponding to the photoplethysmography pulse wave includes:

acquiring multiple trough coordinates in the alternating-current signal, and performing curve fitting on the trough coordinates to obtain a fitting signal;

calculating a difference between the alternating-current signal and the fitting signal to obtain a target alternating-current signal;

averaging multiple peak intensities in the target alternating-current signal to obtain the alternating-current intensity corresponding to the photoplethysmography pulse wave.

In an embodiment, before comparing the fitting bias with the bias range corresponding to the target object further includes:

acquiring multiple historical time periods of the target object and multiple intensity sets corresponding to each historical time period;

for each historical time period, performing curve fitting on the intensity sets corresponding to the historical time period to obtain a historical fitting bias;

obtaining, on a basis of multiple historical fitting biases, the bias range corresponding to the target object.

In an embodiment, after comparing the fitting bias with the bias range corresponding to the target object further includes:

in a situation when it is determined that the fitting bias is within the bias range, taking the fitting coefficient as the blood glucose concentration of the target object in the target time period.

In a second aspect, the present application further provides an apparatus for analyzing a blood glucose concentration. The apparatus includes:

an acquiring module, configured to acquire multiple intensity sets respectively corresponding to multiple photoplethysmography pulse wave sets for a target object in a target time period; the intensity sets include an average alternating-current intensity and an average direct-current intensity;

a fitting module, configured to perform curve fitting on the intensity sets to obtain a fitting coefficient and a fitting bias;

a comparing module, configured to compare the fitting bias with a bias range corresponding to the target object, and in a situation when it is determined that the fitting bias is not within the bias range, adjust the fitting bias to obtain an adjusted fitting bias;

an adjusting module, configured to perform, on a basis of the adjusted fitting bias, curve fitting on the intensity sets to obtain an adjusted fitting coefficient, and take the adjusted fitting coefficient as the blood glucose concentration of the target object in the target time period.

In a third aspect, the present application further provides an electronic device, including a memory and a processor. The memory stores a computer program. The processor implements the steps of the method for analyzing the blood glucose concentration provided in the aforementioned aspects when executing the computer program.

In a fourth aspect, the present application further provides a computer-readable storage medium. The readable storage medium has a computer program stored thereon. The computer program implements the steps of the method for analyzing the blood glucose concentration provided in the aforementioned aspects when executed by the processor.

In a fifth aspect, the present application further provides a computer program product. The computer program product includes a computer program. The computer program implements the steps of the method for analyzing the blood glucose concentration provided in the aforementioned aspects when executed by the processor.

The method and the apparatus for analyzing the blood glucose concentration, the electronic device, and the storage medium, and the computer program product, by performing the curve fitting on the intensity sets, obtain the fitting coefficient. The fitting coefficient reflects a ratio between the alternating-current signal intensity and the direct-current signal intensity in the target time period, that is, the fitting coefficient reflects the blood glucose concentration of the target object in the target time period. Next, the fitting bias is compared with the bias range corresponding to the target object. The bias range characterizes the individual difference of the target object. Different target objects correspond to different bias ranges. If the fitting bias is not within the bias range, which indicates that the fitting bias does not conform to the individual difference of the target object, then the fitting bias is adjusted, so that the obtained adjusted fitting bias conforms to the bias range of the target object. Subsequently, the curve fitting is performed again by taking the adjusted fitting bias and the intensity sets. The obtained adjusted fitting coefficient combines the individual difference of the target object. The adjusted fitting coefficient is taken as the blood glucose concentration of the target object in the target time period, thereby improving the accuracy of the blood glucose concentration.

## Brief description of the drawings

FIG. 1 is an application environment diagram of a method for analyzing a blood glucose concentration in an embodiment;
FIG. 2 is a flowchart of a method for analyzing a blood glucose concentration in an embodiment;
FIG. 3 is a flowchart of steps of correcting a blood glucose concentration in an embodiment;
FIG. 4 is a flowchart of intensity sets acquiring steps in an embodiment;
FIG. 5 is a flow schematic diagram of steps of filtering in an embodiment;
FIG. 6 is a schematic diagram of a method of a photoplethysmography pulse wave in an embodiment;
FIG. 7 is a flowchart of analyzing a blood glucose concentration in an embodiment;
FIG. 8 is a schematic diagram of curves of a blood glucose concentration in an embodiment;
FIG. 9 is a structural block diagram of an apparatus for analyzing a blood glucose concentration in an embodiment;
FIG. 10 is an internal structural diagram of an electronic device in an embodiment.

## Description of the embodiments

In order to make the objectives, technical solutions, and advantages of the present application clearer, the present application is described in further detail below in combination with the accompanying drawings and embodiments. It should be understood that the specific embodiments described herein are only used to explain the present application and are not used to limit the present application.

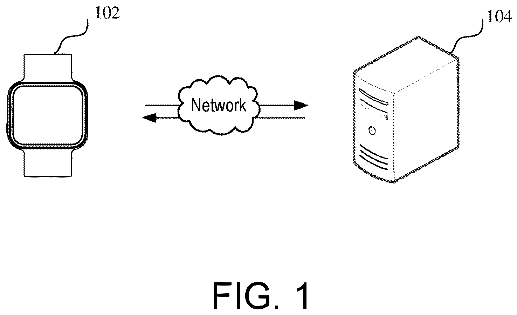

A method for analyzing a blood glucose concentration provided by the embodiments of the present application may be applied in an application environment as shown in FIG. 1. A device 102 communicates with a server 104 through a network. A data storage system may store data that the server 104 needs to process. The data storage system may be integrated on the server 104, or may be placed on the cloud or other network servers. Both the device and the server may be individually configured to execute the method for analyzing the blood glucose concentration provided in the embodiments of the present application. The device and the server may also be cooperatively configured to execute the method for analyzing the blood glucose concentration provided in the embodiments of the present application. For example, the device acquires intensity sets corresponding to multiple photoplethysmography pulse wave sets for a target object in a target time period, performs curve fitting on the intensity sets to obtain a fitting coefficient and a fitting bias, compares the fitting bias with a bias range corresponding to the target object, adjusts the fitting bias to obtain an adjusted fitting bias when determining that the fitting bias is not within the bias range, performs, on the basis of the adjusted fitting bias, curve fitting on the intensity sets to obtain an adjusted fitting coefficient, and takes the adjusted fitting coefficient as the blood glucose concentration of the target object in the target time period. The device 102 may be, but is not limited to, various portable wearable devices. The portable wearable device may be a smart watch, a smart bracelet, and a head-mounted devices. The server 104 may be implemented by an independent server or a server cluster composed of multiple servers.

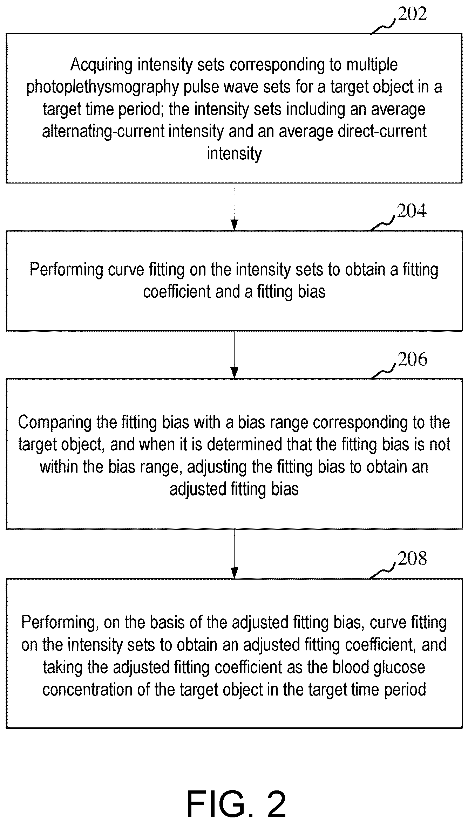

In an embodiment, as shown in FIG. 2, the method for analyzing the blood glucose concentration is provided, including step 202 to step 208. The method is described by taking an application to the device as an example.

In step 202, the intensity sets corresponding to the photoplethysmography pulse wave sets for the target object are acquired in the target time period. The intensity sets include an average alternating-current intensity and an average direct-current intensity.

The target object refers to an individual whose blood glucose concentration is monitored. For example, the individual wears the device. The target time period refers to a time period for monitoring the blood glucose concentration. The duration of the target time period may be set according to actual requirements. The photoplethysmography pulse wave refers to a light wave formed by using light of a specific frequency to irradiate the skin surface and receiving transmitted light or reflected light. Different components in blood have different degrees of absorption for light of different frequencies. For example, blood glucose in blood has strong absorption capability for light of frequency A, and changes in the blood glucose concentration may directly affect the absorption of light of frequency A, which causes changes in transmitted light or reflected light, that is, causing changes in the photoplethysmography pulse wave. A photoplethysmography pulse wave set refers to a set composed of multiple photoplethysmography pulse waves corresponding to emitted light of the same frequency and same intensity. It may be understood that, when the emitted light has the same frequency and same intensity, each reflected light wave or transmitted light wave of a preset duration that is received serves as one photoplethysmography pulse wave. The photoplethysmography pulse waves form one photoplethysmography pulse wave set. For example, the device emits light of frequency A and intensity B for 30 seconds, receives reflected light waves for 30 seconds, uses each 10-second reflected light wave as one photoplethysmography pulse wave, and obtains three photoplethysmography pulse waves within 30 seconds. The threee photoplethysmography pulse waves form one photoplethysmography pulse wave set. An intensity set refers to a set composed of the average direct-current intensity of the photoplethysmography pulse waves and the average alternating-current intensity of the photoplethysmography pulse waves.

Exemplarily, in order to monitor the blood glucose concentration of the target object in the target time period, the device acquires the intensity sets of the target object in the target time period. Each intensity set is obtained by analyzing one photoplethysmography pulse wave set.

In step 204, the curve fitting is performed on the intensity sets to obtain the fitting coefficient and the fitting bias.

The curve fitting refers to a process of selecting an appropriate curve type to fit the intensity sets. It may be understood that the average direct-current intensity of the intensity sets is taken as an X-axis coordinate value, the average alternating-current intensity is taken as a Y-axis coordinate value, and the intensity set is taken as one coordinate in a rectangular coordinate system to perform the curve fitting on multiple coordinates. The curve fitting includes but is not limited to partial least squares fitting and linear fitting. The fitting coefficient refers to a coefficient of the fitting curve. The fitting bias refers to an intercept of the fitting curve. For example, the fitting curve fitted by using the intensity sets is $Y=KX+B$, where K is the fitting coefficient, and B is the fitting bias.

Exemplarily, the device performs the curve fitting on the intensity sets to obtain an initial fitting curve, takes a coefficient of the initial fitting curve as the fitting coefficient, and takes an intercept of the initial fitting curve as the fitting bias.

In step 206, the fitting bias is compared with the bias range corresponding to the target object. When it is determined that the fitting bias is not within the bias range, the fitting bias is adjusted to obtain the adjusted fitting bias.

The bias range refers to an intercept range corresponding to the target object. Different target objects correspond to different bias ranges. The bias range characterizes the individual differences of the target object.

Exemplarily, the device acquires the bias range corresponding to the target object, and compares the fitting bias with the bias range. If the fitting bias is within the bias range, the fitting coefficient is taken as the blood glucose concentration of the target object in the target time period. If the fitting bias is not within the bias range, the fitting bias is adjusted to obtain the adjusted fitting bias. The adjusted fitting bias is adjusted within the bias range.

In an embodiment, adjusting the fitting bias to obtain the adjusted fitting bias includes: performing, on the bias range, multiple adjustments on the fitting bias to obtain multiple candidate fitting biases; and for each candidate fitting bias, performing curve fitting on the intensity sets to obtain a candidate fitting curve; calculating the linearity of the candidate fitting curve; comparing the linearity of the candidate fitting curves; and determining the candidate fitting bias of the candidate fitting curve corresponding to the maximum linearity as the adjusted fitting bias.

In step 208, on the basis of the adjusted fitting bias, the curve fitting is performed on the intensity sets to obtain the adjusted fitting coefficient. The adjusted fitting coefficient is taken as the blood glucose concentration of the target object in the target time period.

**EP 4 702 921 A1**

The blood glucose concentration refers to the concentration of glucose in the blood.

Exemplarily, the device performs the curve fitting on the intensity sets according to the adjusted fitting bias to obtain the adjusted fitting curve, takes the coefficient of the adjusted fitting curve as the adjusted fitting coefficient, and takes the adjusted fitting coefficient as the blood glucose concentration of the target object in the target time period.

In the method for analyzing the blood glucose concentration, by performing the curve fitting on the intensity sets, the fitting coefficient is obtained. The fitting coefficient reflects a ratio between an alternating-current signal intensity and a direct-current signal intensity in the target time period, that is, the blood glucose concentration of the target object is reflected in the target time period. Next, the fitting bias is compared with the bias range corresponding to the target object. The bias range characterizes the individual differences of the target object. Different target objects correspond to different bias ranges. If the fitting bias is not within the bias range, which indicates that the fitting bias does not conform to the individual differences of the target object, then the fitting bias is adjusted, so that the obtained adjusted fitting bias conforms to the bias range of the target object. Subsequently, the curve fitting is performed again by taking the adjusted fitting bias and the intensity sets. The obtained adjusted fitting coefficient combines the individual differences of the target object. The adjusted fitting coefficient is taken as the blood glucose concentration of the target object in the target time period, thereby improving the accuracy of the blood glucose concentration.

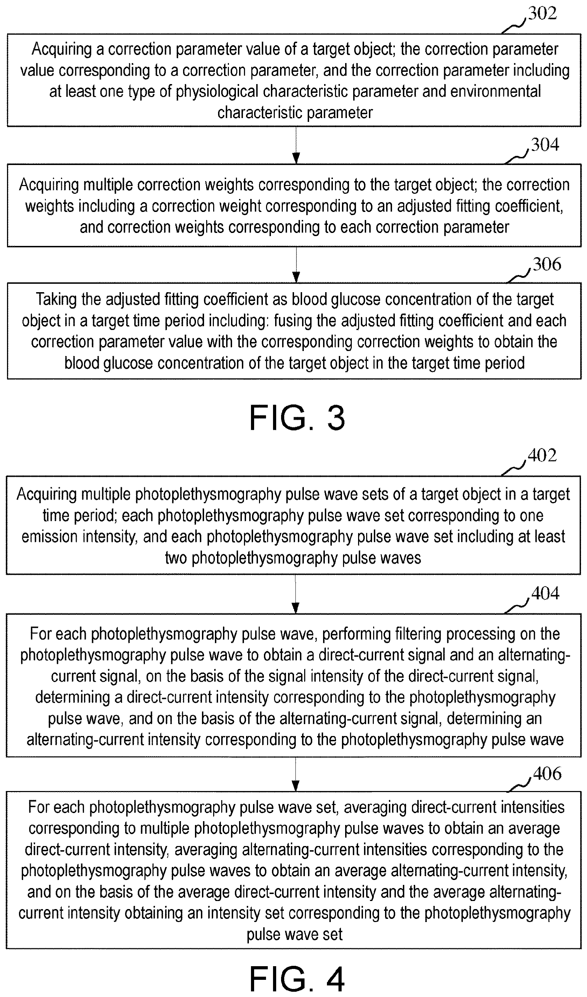

In an embodiment, as shown in FIG. 3, step 208, on the basis of the adjusted fitting bias, the curve fitting is performed on the intensity sets to obtain the adjusted fitting coefficient, and thereafter further includes:
In step 302, a correction parameter value of the target object is acquired; the correction parameter value corresponds to a correction parameter, and the correction parameter includes at least one type of physiological characteristic parameter and environmental characteristic parameter.

The correction parameter refers to a parameter for correcting the blood glucose concentration. It may be understood as a parameter that affects the blood glucose concentration. The correction parameter value refers to the specific numerical value of the correction parameter at a certain moment. The physiological characteristic parameter refers to a characteristic parameter that characterizes a physical condition of the target object. The physiological characteristic parameter includes but is not limited to blood pressure, heart rate, blood oxygen, and body mass index (BMI). The environmental characteristic parameter refers to a characteristic parameter that characterizes an environmental condition. The environmental characteristic parameter includes but is not limited to temperature and humidity.

Exemplarily, after the adjusted fitting coefficient is obtained, in order to further improve the accuracy of the monitored blood glucose concentration, the adjusted fitting coefficient is corrected in combination with the physiological characteristic parameters and/or environmental characteristic parameters of the target object, and the corrected result is taken as the blood glucose concentration of the target object in the target time period. A sensor on the device acquires the correction parameter value of the target object.

In step 304, multiple correction weights corresponding to the target object are acquired; the correction weights include a correction weight corresponding to the adjusted fitting coefficient and correction weights corresponding to each correction parameter.

The correction weight refers to a proportion corresponding to the correction parameter. The larger the correction weight corresponding to the correction parameter, the greater the influence of the correction parameter on the blood glucose concentration. The correction weight has individual differences, which may be understood as different objects having different correction weights corresponding to the same correction parameter.

Exemplarily, the device acquires the correction weights of the target object from a database.

In step 306, taking the adjusted fitting coefficient as the blood glucose concentration of the target object in the target time period includes: fusing the adjusted fitting coefficient and each correction parameter value with the corresponding correction weights to obtain the blood glucose concentration of the target object in the target time period.

Exemplarily, the device multiplies the adjusted fitting coefficient with the corresponding correction weight to obtain a corrected fitting coefficient, multiplies each correction parameter value with the corresponding correction weight to obtain corresponding multiple correction values, and adds the corrected fitting coefficient with the correction values to obtain the blood glucose concentration of the target object in the target time period.

In this embodiment, after performing, on the basis of the adjusted fitting bias, the curve fitting on the intensity sets to obtain the adjusted fitting coefficient, the device acquires correction parameter values that affect blood glucose concentration and correction weights corresponding to the target object. Different target objects have different correction weights corresponding to the same correction parameter. The correction weights reflect the individual differences of the target object. By combining the correction parameter values that affect the blood glucose concentration and the corresponding correction weights to correct the adjusted fitting coefficient, it may be understood that the blood glucose concentration of the target object in the target time period is comprehensively analyzed while considering the physical characteristics of the target object and the environmental characteristics of the environment where the target object is located, further improving the accuracy of the blood glucose concentration.

In step 202 of an embodiment, as shown in FIG. 4, acquiring the intensity sets corresponding to the photoplethysmography pulse wave sets for the target object in the target time period, includes:

In step 402, the photoplethysmography pulse wave sets of the target object are acquired in the target time period; each photoplethysmography pulse wave set corresponds to one emission intensity, and each photoplethysmography pulse wave set includes at least two photoplethysmography pulse waves.

The emission intensity refers to the intensity of light waves emitted by the device. The emission intensity may be achieved by adjusting the peak intensity value of the emitted light, or may be achieved by adjusting the proportion of the light emission duration.

Exemplarily, in order to monitor the blood glucose concentration of the target object in the target time period, the device acquires the photoplethysmography pulse waves of the target object in the target time period, groups the photoplethysmography pulse waves corresponding to the same emission intensity into one photoplethysmography pulse wave set, and obtains the photoplethysmography pulse wave sets.

In step 404, for each photoplethysmography pulse wave, filtering processing is performed on the photoplethysmography pulse wave to obtain a direct-current signal and an alternating-current signal. On the basis of the signal intensity of the direct-current signal, the direct-current intensity corresponding to the photoplethysmography pulse wave is determined. On the basis of the alternating-current signal, the alternating-current intensity corresponding to the photoplethysmography pulse wave is determined.

The filtering processing refers to separating the direct-current signal and the alternating-current signal of the photoplethysmography pulse wave based on preset rules. The alternating-current signal refers to the alternating-current component of the photoplethysmography pulse wave, which is synchronized with the heart rate and reflects the light absorption of arterial blood. The direct-current signal refers to the direct-current component of the photoplethysmography pulse wave, which reflects the light absorption of non-arterial blood, venous blood, capillary blood, and muscle tissue.

Exemplarily, for each photoplethysmography pulse wave, the device, on the basis of a preset frequency range, separates the direct-current signal and the alternating-current signal from the photoplethysmography pulse wave, takes the signal intensity of the direct-current signal as the direct-current intensity of the photoplethysmography pulse wave, and on the basis of the peak intensity and trough intensity of the alternating-current signal, determines the alternating-current intensity corresponding to the photoplethysmography pulse wave.

In an embodiment, on the basis of the alternating-current signal, determining the alternating-current intensity corresponding to the photoplethysmography pulse wave includes: the device acquires the peak intensity corresponding to each peak in the alternating-current signal and a trough intensity corresponding to each trough, adds multiple peak intensities to obtain a peak statistical intensity, adds multiple trough intensities to obtain a trough statistical intensity, counts a number of peaks or troughs to obtain a statistical value, calculates a difference value between the peak statistical intensity and the trough statistical intensity, and divides the difference value by the statistical value to obtain the alternating-current intensity corresponding to the photoplethysmography pulse wave.

In step 406, for each photoplethysmography pulse wave set, the direct-current intensities corresponding to the photoplethysmography pulse waves are averaged to obtain an average direct-current intensity, the alternating-current intensities corresponding to the photoplethysmography pulse waves are averaged to obtain an average alternating-current intensity, and on the basis of the average direct-current intensity and the average alternating-current intensity, the intensity set corresponding to the photoplethysmography pulse wave set is obtained.

Exemplarily, the device averages the direct-current intensities corresponding to the photoplethysmography pulse waves of the same photoplethysmography pulse wave set to obtain the average direct-current intensity, averages the alternating-current intensities corresponding to multiple photoplethysmography pulse waves to obtain the average alternating-current intensity, and composes the intensity set corresponding to the photoplethysmography pulse wave set with the average direct-current intensity and the average alternating-current intensity.

In this embodiment, by composing the intensity set for curve fitting with the average direct-current intensity and the average alternating-current intensity, fluctuations or measurement errors of one photoplethysmography pulse wave that may cause inaccuracy of the intensity set and further cause inaccuracy of a curve fitting result are avoided, thereby improving the accuracy of the intensity set, and providing accurate basic data for subsequent curve fitting.

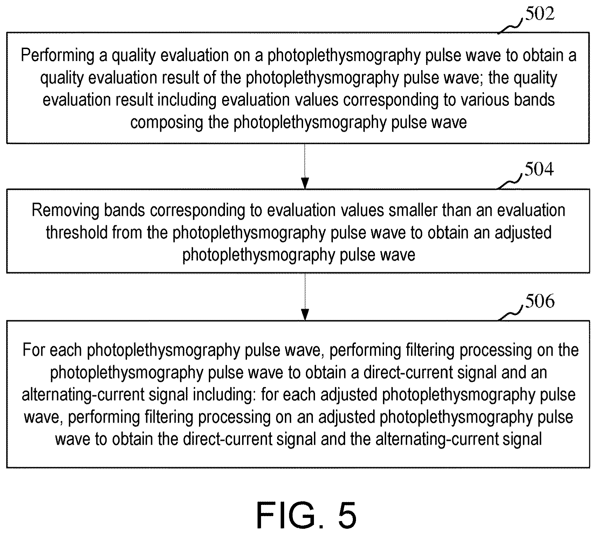

In step 404 of an embodiment, as shown in FIG. 5, for each photoplethysmography pulse wave, before performing the filtering processing on the photoplethysmography pulse wave to obtain the direct-current signal and the alternating-current signal, further includes:

In step 502, a quality evaluation is performed on the photoplethysmography pulse wave to obtain a quality evaluation result of the photoplethysmography pulse wave; the quality evaluation result includes evaluation values corresponding to various bands composing the photoplethysmography pulse wave.

The quality evaluation refers to evaluating the quality of the photoplethysmography pulse wave. It may be understood as evaluating a waveform of the photoplethysmography pulse wave. The quality evaluation result refers to a set composed of evaluation values corresponding to various bands of the photoplethysmography pulse wave. The preset band refers to a standard band that is preset and configured to evaluate bands. The preset band may be set according to experience or experimental data.

Exemplarily, in the aforementioned step 404, the filtering processing is directly performed on the photoplethysmography pulse wave. The photoplethysmography pulse wave may have a large error and may not reflect the real situation of the target object. In order to improve the accuracy of the intensity set, before performing the filtering processing on the photoplethysmography pulse wave, it is necessary to perform preprocessing on the photoplethysmography pulse wave. The steps of preprocessing include steps 502 and 504. The device divides the photoplethysmography pulse wave into multiple bands, calculates the similarity between each band and a preset band to obtain an evaluation value of the band, and composes an evaluation result of the photoplethysmography pulse wave with the evaluation values of the bands.

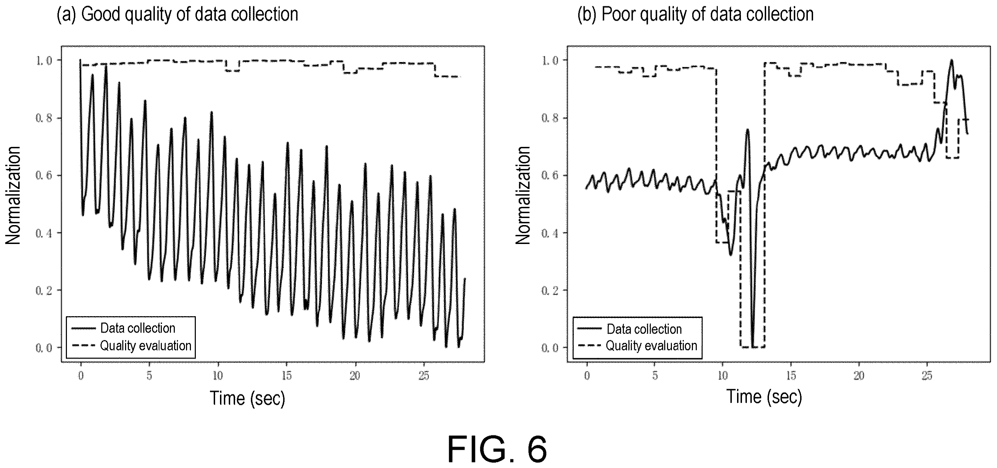

In step 502 of an embodiment, performing the quality evaluation on the photoplethysmography pulse wave to obtain the quality evaluation result of the photoplethysmography pulse wave, includes: performing normalization processing on the photoplethysmography pulse wave to obtain a photoplethysmography pulse wave to be evaluated, as shown in the collected data in FIG. 6, dividing the photoplethysmography pulse wave to be evaluated into the bands, for each band, calculating the similarity between the band and the preset band to obtain an initial evaluation value of the band, performing normalization processing on the initial evaluation value to obtain an evaluation value of the band, and composing the evaluation result of the photoplethysmography pulse wave with the evaluation values of the bands, as shown in the quality evaluation in FIG. 6.

In step 504, bands corresponding to evaluation values smaller than an evaluation threshold are removed from the photoplethysmography pulse wave to obtain an adjusted photoplethysmography pulse wave.

The evaluation threshold refers to the minimum evaluation value for retaining bands. The evaluation threshold may be set according to actual requirements.

Exemplarily, the device respectively compares each evaluation value with the evaluation threshold, and removes the bands corresponding to the evaluation values smaller than the evaluation threshold from the photoplethysmography pulse wave to obtain the adjusted photoplethysmography pulse wave. In step 504 of an embodiment, before removing the bands corresponding to the evaluation values smaller than the evaluation threshold from the photoplethysmography pulse wave to obtain the adjusted photoplethysmography pulse wave, further includes: the device respectively compares each evaluation value of the photoplethysmography pulse wave with the evaluation threshold, and counts the proportion of evaluation values smaller than the evaluation threshold of the quality evaluation result. If the proportion is greater than a preset threshold value, then the device determines that the quality of the photoplethysmography pulse wave is poor and deletes the photoplethysmography pulse wave. If the proportion is smaller than or equal to the preset threshold value, then the device determines that the quality of the photoplethysmography pulse wave is good, and removes the bands corresponding to the evaluation values smaller than the evaluation threshold from the photoplethysmography pulse wave to obtain the adjusted photoplethysmography pulse wave. For example, the photoplethysmography pulse wave in FIG. 6(a) has good quality, and then the bands corresponding to the evaluation values smaller than the evaluation threshold are removed from the photoplethysmography pulse wave to obtain the adjusted photoplethysmography pulse wave. Having poor quality in FIG. 6(b), the photoplethysmography pulse wave is deleted from the photoplethysmography pulse wave set.

In step 506, for each photoplethysmography pulse wave, the filtering processing is performed on the photoplethysmography pulse wave to obtain the direct-current signal and the alternating-current signal includes: for each adjusted photoplethysmography pulse wave, performing the filtering processing on the adjusted photoplethysmography pulse wave to obtain the direct-current signal and the alternating-current signal.

Exemplarily, after obtaining the adjusted photoplethysmography pulse wave, for each adjusted photoplethysmography pulse wave, the device separates the direct-current signal and the alternating-current signal from the adjusted photoplethysmography pulse wave according to a preset frequency range.

In this embodiment, the quality evaluation is performed on the photoplethysmography pulse wave. The bands corresponding to the evaluation values smaller than the evaluation threshold are removed from the photoplethysmography pulse wave. When the evaluation value is smaller than the evaluation threshold, it indicates that the band corresponding to the evaluation value is a mutation band with large differences from other bands. The mutation band may not accurately reflect the normal variation of reflected light or transmitted light. Removing this band from the photoplethysmography pulse wave may be understood as removing the mutation band from the photoplethysmography pulse wave. The adjusted photoplethysmography pulse wave may more accurately reflect the variation of reflected light or transmitted light, improving the accuracy of the adjusted photoplethysmography pulse wave, and thereby improving the accuracy of the separated direct-current signal and the separated alternating-current signal.

In step 404 of an embodiment, on the basis of the alternating-current signal, determining the alternating-current intensity corresponding to the photoplethysmography pulse wave includes:

Multiple trough coordinates in the alternating-current signal are acquired, the curve fitting is performed on the trough coordinates to obtain a fitting signal; a difference between the alternating-current signal and the fitting signal is calculated to obtain a target alternating-current signal; and multiple peak intensities in the target alternating-current signal are averaged to obtain the alternating-current intensity corresponding to the photoplethysmography pulse wave.

The trough coordinate refers to a coordinate of the lowest point in a trough of the alternating-current signal. One dimension of the trough coordinate is a time dimension, and the other dimension is an intensity dimension. For example, as shown in FIG. 6(a), a first trough coordinate is (0, 0.45). The fitting signal refers to a fitting curve obtained by fitting the trough coordinates. The peak intensity refers to an intensity of the highest point of the peak in the target alternating-current signal.

Exemplarily, after obtaining the alternating-current signal of the photoplethysmography pulse wave, the device acquires a trough coordinate of each trough in the alternating-current signal, performs the curve fitting on the trough coordinates to obtain the fitting signal, subtracts the fitting signal from the alternating-current signal to obtain the target alternating-current signal, acquires the peak intensity of each peak in the target alternating-current signal, and averages the peak intensities to obtain the alternating-current intensity corresponding to the photoplethysmography pulse wave.

In this embodiment, compared to obtaining the peak intensity to be averaged by directly using the peak intensity minus the trough intensity, the peak intensity to be averaged is affected by time, that is, the subtracted peak intensity and trough intensity are not at the same time. The peak intensity to be averaged includes the influence of time factors, and has low accuracy. The alternating-current signal minus the fitting signal is used to obtain the target alternating-current signal. The target alternating-current signal reflects an amplitude of the alternating-current signal variation. The peak intensity to be averaged is acquired from the target alternating-current signal. The peak intensity is not affected by time factors, improving the accuracy of the peak intensity to be averaged, and thereby improving the accuracy of the alternating-current intensity.

In step 206 of an embodiment, before comparing the fitting bias with the bias range corresponding to the target object, further includes:
Multiple historical time periods of the target object and the intensity sets corresponding to each historical time period are acquired; for each historical time period, the curve fitting is performed on the intensity sets corresponding to the historical time period to obtain a historical fitting bias; and on the basis of multiple historical fitting biases, the bias range corresponding to the target object is obtained.

The historical time period refers to a previous time period compared to the current time.

Exemplarily, before starting to monitor the blood glucose concentration of the target object, the device needs to determine the bias range corresponding to the target object. The device may acquire the historical time periods from a wearing time period after the target object has worn the device for a period of time, determine a bias range that conforms to the individual differences of the target object according to the intensity sets corresponding to the historical time periods, then save the bias range and the target object identifier of the target object, and take the bias range in the subsequent process of monitoring the blood glucose concentration of the target object. Alternatively, before monitoring of the blood glucose concentration of the target object every time, the device acquires the intensity sets corresponding to the historical time periods before the target time period, and determines the bias range according to the intensity sets. The device acquires the historical time periods of the target object, acquires the intensity sets corresponding to each historical time period, performs the curve fitting on the intensity sets for each historical time period to obtain the historical fitting bias, averages the historical fitting biases to obtain an average bias, and on the basis of the average bias and a preset floating range, obtains the bias range corresponding to the target object. For example, if the average bias is M and the preset floating range is plus or minus 10%, then the bias range is [M*(1+10%), M*(1-10%)].

In this embodiment, by fitting the intensity sets corresponding to each historical time period of the target object to obtain multiple fitting biases, and determining the bias range corresponding to the target object according to the fitting biases, the bias range corresponds to the target object one by one, has individual differences, and may more accurately characterize the individual differences of the target object.

In step 206 of an embodiment, after comparing the fitting bias with the bias range corresponding to the target object, further includes:
In a situation when it is determined that the fitting bias is within the bias range, the fitting coefficient is taken as the blood glucose concentration of the target object in the target time period.

Exemplarily, in step 206, the device compares the fitting bias with the bias range corresponding to the target object. If the fitting bias is within the bias range, then the fitting coefficient is taken as the blood glucose concentration of the target object in the target time period.

In this embodiment, if the fitting bias is within the bias range, it indicates that the fitting bias obtained through the curve fitting conforms to the individual differences of the target object, and the fitting coefficient has high accuracy, which may accurately reflect the blood glucose concentration of the target object in the target time period.

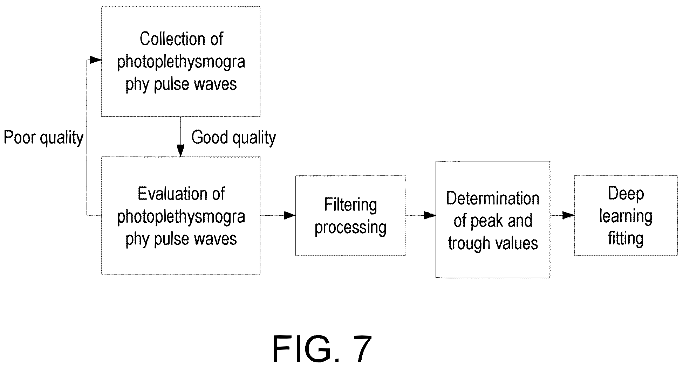

In an exemplary embodiment, a process of analyzing the blood glucose concentration is shown in FIG. 7. The device emits light of A frequency and first intensity, receives photoplethysmography pulse waves for a preset duration, performs normalization processing on the photoplethysmography pulse waves to obtain photoplethysmography pulse waves to be evaluated, divides the photoplethysmography pulse waves to be evaluated into the bands, for each band, calculates the similarity between the band and a preset band to obtain an initial evaluation value of the band, performs normalization processing on the initial evaluation value to obtain an evaluation value of the band, composes the evaluation values of the bands into an evaluation result of the photoplethysmography pulse wave, on the basis of the evaluation result, and determines the quality of the photoplethysmography pulse wave. If the quality of the photoplethysmography pulse wave is poor, then the device deletes the photoplethysmography pulse wave. If the quality of the photoplethysmography pulse wave is good, then the device removes the bands corresponding to the evaluation values less than the evaluation threshold from the photoplethysmography pulse wave to obtain adjusted photoplethysmography pulse waves. The aforementioned process is repeated to obtain a preset number of photoplethysmography pulse waves. The preset number of photoplethysmography pulse waves compose a first photoplethysmography pulse wave set. The device adjusts the intensity of emitted light to a second intensity, emits light of A frequency and second intensity, repeats the aforementioned process to obtain a preset number of photoplethysmography pulse waves. The preset number of photoplethysmography pulse waves compose a second photoplethysmography pulse wave set. The device adjusts the intensity of emitted light to a third intensity, emits light of A frequency and third intensity, repeats the aforementioned process to obtain a preset number of photoplethysmography pulse waves. The preset number of photoplethysmography pulse waves compose a third photoplethysmography pulse wave set.

For each photoplethysmography pulse wave of each photoplethysmography pulse wave set, on the basis a preset frequency range, the filtering processing is performed on the photoplethysmography pulse wave to separate the direct-current signal and the alternating-current signal from the photoplethysmography pulse wave.

The signal intensity of the direct-current signal serves as the direct-current intensity of the photoplethysmography pulse wave. The trough coordinates of each trough in the alternating-current signal are obtained. The curve fitting is performed on the trough coordinates to obtain the fitting signal. The alternating-current signal minus the fitting signal obtains the target alternating-current signal. The peak intensity of each peak in the target alternating-current signal is acquired. Averaging is performed on the peak intensities to obtain the alternating-current intensity corresponding to the photoplethysmography pulse wave. For each photoplethysmography pulse wave set, averaging is performed on the direct-current intensities corresponding to the photoplethysmography pulse waves to obtain the average direct-current intensity, averaging is performed on the alternating-current intensities corresponding to the photoplethysmography pulse waves to obtain the average alternating-current intensity. On the basis of the average direct-current intensity and the average alternating-current intensity, the intensity set corresponding to the photoplethysmography pulse wave set is obtained. The average direct-current intensity and the average alternating-current intensity compose the intensity set corresponding to the photoplethysmography pulse wave set.

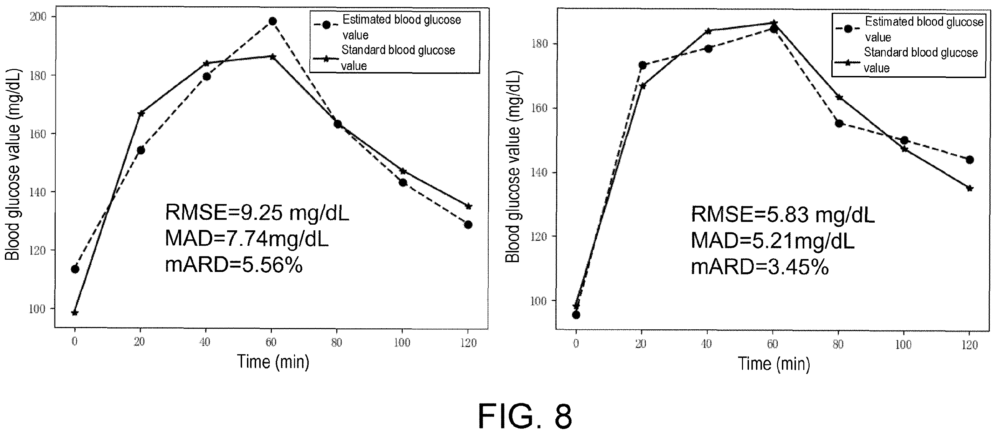

The curve fitting is performed on the intensity sets to obtain the initial fitting curve. The coefficient of the initial fitting curve serves as the fitting coefficient, and the intercept of the initial fitting curve serves as a fitting bias to obtain the bias range corresponding to the target object. The fitting bias is compared with the bias range. If the fitting bias is within the bias range, the fitting coefficient serves as the blood glucose concentration of the target object in the target time period. If the fitting bias is not within the bias range, the fitting bias is adjusted to obtain the adjusted fitting bias. The adjusted fitting bias is within the bias range, and according to the adjusted fitting bias, the curve fitting is performed on the intensity sets to obtain an adjusted fitting curve. The coefficient of the adjusted fitting curve serves as an adjusted fitting coefficient, the adjusted fitting coefficient serves as the blood glucose concentration of the target object in the target time period, and multiple initial blood glucose concentrations in continuous time periods are connected into a curve to achieve continuous monitoring of blood glucose concentration changes of the target object. As shown in the left figure in FIG. 8, the units of an estimated blood glucose value and a standard blood glucose value are both mg/dL (concentration unit, milligrams per deciliter). Each point in a curve of the estimated blood glucose value represents the blood glucose concentration of the target object in the target time period. The method for analyzing the blood glucose concentration is adopted to obtain that the estimated blood glucose value represents the change situation of the blood glucose concentration of the target object, and the standard blood glucose value represents the real blood glucose concentration change situation of the target object. On the basis of preset correction parameters, the device obtains correction parameter values corresponding to the target object and correction weights corresponding to the target object. The adjusted fitting coefficient is multiplied with the corresponding correction weight to obtain a corrected fitting coefficient. Each correction parameter value is multiplied with the corresponding correction weight to obtain corresponding multiple correction values. The corrected fitting coefficient is added with multiple correction values to obtain the blood glucose concentration of the target object in the target time period. As shown in the right figure in FIG. 8, each point in the curve of the estimated blood glucose value represents the blood glucose concentration of the target object in the target time period. The blood glucose concentration combines physiological characteristics and environmental characteristics of the target object. The method for analyzing the blood glucose concentration is adopted to obtain that the estimated blood glucose value represents the change situation of the blood glucose concentration of the target object, and the standard blood glucose value represents the real blood glucose concentration change situation of the target object.

In order to evaluate the accuracy of the method for analyzing the blood glucose concentration, a formula (1) is adopted to calculate a mean absolute relative difference (mARD) between the estimated blood glucose value and the standard blood glucose value shown in FIG. 8, a formula (2) is adopted to calculate a root mean square error (RMSE) between the estimated blood glucose value and the standard blood glucose value shown in FIG. 8, and a formula (3) is adopted to calculate a mean absolute difference (MAD) between the estimated blood glucose value and the standard blood glucose value shown in FIG. 8:

$$mARD = \frac{1}{N}\sum_{t=1}^{N} \frac{|g(t)-\hat{g}(t)|}{g(t)} \qquad \text{formula (1)}$$

$$RMSE = \frac{1}{N}\sum_{t=1}^{N} \sqrt{\left(g(t)-\hat{g}(t)\right)^2} \qquad \text{formula (2)}$$

$$MAD = \frac{1}{N}\sum_{t=1}^{N} |g(t)-\hat{g}(t)| \qquad \text{formula (3)}$$

where, g(t) is a standard blood glucose value, g(t) is a estimated blood glucose value, t represents a time point, N represents a number of the included time points.

By comparing the three indicator results in the left and right figures in FIG. 8, it may be known that the estimated blood glucose value obtained by combining the physiological characteristics and environmental characteristics of the target object is more accurate and closer to the standard blood glucose value than the estimated blood glucose value obtained without combining the physiological characteristics and environmental characteristics of the target object.

It should be understood that, although the steps in the flowcharts involved in the aforementioned embodiments are sequentially shown according to the indication of arrows, these steps are not necessarily executed sequentially in the order indicated by the arrows. Unless explicitly stated herein, the execution of these steps does not have strict order restrictions, and these steps may be executed in other orders. Moreover, at least a portion of the steps in the flowcharts involved in the aforementioned embodiments may include multiple steps or multiple stages, and these steps or stages are not necessarily executed and completed at the same time, but may be executed at different times, and the execution order of these steps or stages is not necessarily sequential, but may be executed alternately or in rotation with at least a portion of other steps or steps or stages in other steps.

On the basis of the same inventive concept, the embodiments of the present application also provide an apparatus for analyzing a blood glucose concentration for implementing the method for analyzing the blood glucose concentration. The implementation solution for solving problems provided by the device is similar to the implementation solution recorded in the method, so the specific limitations in one or multiple embodiments of the apparatus for analyzing the blood glucose concentration provided below may refer to the limitations in the method for analyzing the blood glucose concentration, which is not repeated here.

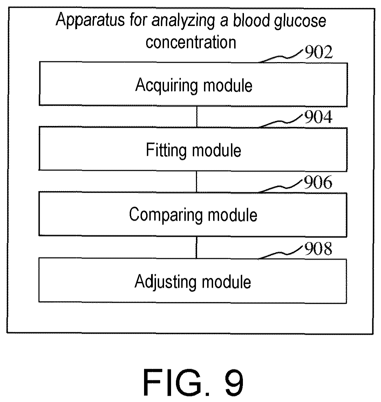

In an embodiment, as shown in FIG. 9, the apparatus for analyzing the blood glucose concentration is provided, including: an acquiring module 902, a fitting module 904, a comparing module 906, and an adjusting module 908, where: The acquiring module 902 is configured to acquire the intensity sets respectively corresponding to the photoplethysmography pulse wave sets for the target object in the target time period. The intensity set includes the average alternating-current intensity and the average direct-current intensity.

The fitting module 904 is configured to perform the curve fitting on the intensity sets to obtain the fitting coefficient and the fitting bias.

The comparing module 906 is configured to compare the fitting bias with the bias range corresponding to the target object, and when it is determined that the fitting bias is not within the bias range, adjust the fitting bias to obtain the adjusted fitting bias.

The adjusting module 908 is configured to, on the basis of the adjusted fitting bias, perform the curve fitting on the intensity sets to obtain the adjusted fitting coefficient, and take the adjusted fitting coefficient as the blood glucose concentration of the target object in the target time period.

In an embodiment, the apparatus for analyzing the blood glucose concentration further includes a correction module. The correction module is configured to: acquire correction parameter values of the target object; the correction parameter values correspond to correction parameters, and the correction parameters include at least one type of physiological characteristic parameters and environmental characteristic parameters; and acquire multiple correction weights corresponding to the target object; the multiple correction weights include correction weights corresponding to the adjusted fitting coefficient, and correction weights corresponding to each correction parameter. Taking the adjusted fitting coefficients as the blood glucose concentration of the target object in the target time period includes: fusing the adjusted fitting coefficient and each correction parameter value with corresponding correction weights to obtain the blood glucose concentration of the target object in the target time period.

In an embodiment, the acquiring module 902 is further configured to: acquire the photoplethysmography pulse wave sets for the target object in the target time period; each photoplethysmography pulse wave set corresponds to one emission intensity, each photoplethysmography pulse wave set includes at least two photoplethysmography pulse waves; for each photoplethysmography pulse wave, perform the filtering processing on the photoplethysmography pulse wave to obtain the direct-current signal and the alternating-current signal; on the basis of the signal intensity of the direct-current signal, determine the direct-current intensity corresponding to the photoplethysmography pulse wave; on the basis of the alternating-current signal, determine the alternating-current intensity corresponding to the photoplethysmography pulse wave; for each photoplethysmography pulse wave set, average the direct-current intensities corresponding to the photoplethysmography pulse waves to obtain the average direct-current intensity, average the alternating-current intensities corresponding to the photoplethysmography pulse waves to obtain the average alternating-current intensity, and on the basis of the average direct-current intensity and average alternating-current intensity, obtain the intensity set corresponding to the photoplethysmography pulse wave set.

In an embodiment, the apparatus for analyzing the blood glucose concentration further includes an evaluation module. The evaluation module is configured to: perform the quality evaluation on the photoplethysmography pulse wave to obtain the quality evaluation result of the photoplethysmography pulse wave; the quality evaluation result includes evaluation values corresponding to each band composing the photoplethysmography pulse wave; and remove the bands corresponding to the evaluation values less than the evaluation threshold from the photoplethysmography pulse wave to obtain the adjusted photoplethysmography pulse wave. For each photoplethysmography pulse wave, performing the filtering processing on the photoplethysmography pulse wave to obtain the direct-current signal and the alternating-current signal includes: for each adjusted photoplethysmography pulse wave, performing the filtering processing on the adjusted photoplethysmography pulse wave to obtain the direct-current signal and the alternating-current signal.

In an embodiment, the acquiring module 902 is further configured to: acquire the trough coordinates in the alternating-current signal, perform the curve fitting on the trough coordinates to obtain the fitting signal; calculate the difference between the alternating-current signal and the fitting signal to obtain the target alternating-current signal; and average the peak intensities in the target alternating-current signal to obtain the alternating-current intensity corresponding to the photoplethysmography pulse wave.

In an embodiment, the apparatus for analyzing the blood glucose concentration further includes an analysis module. The analysis module is configured to: acquire the historical time periods of the target object, and the intensity sets corresponding to each historical time period; for each historical time period, perform the curve fitting on the intensity sets corresponding to the historical time period to obtain the historical fitting bias; and on the basis of the historical fitting biases, obtain the bias range corresponding to the target object.

In an embodiment, the comparing module 906 is further configured to: when it is determined that the fitting bias is within the bias range, take the fitting coefficient as the blood glucose concentration of the target object in the target time period.

Each module in the apparatus for analyzing the blood glucose concentration may be implemented entirely or partially by a software, a hardware, and a combination thereof. The aforementioned modules may be embedded in or independent of a processor in an electronic device in a hardware form, or may be stored in a memory of the electronic device in software form, so that the processor may call and execute operations corresponding to each of the aforementioned modules.

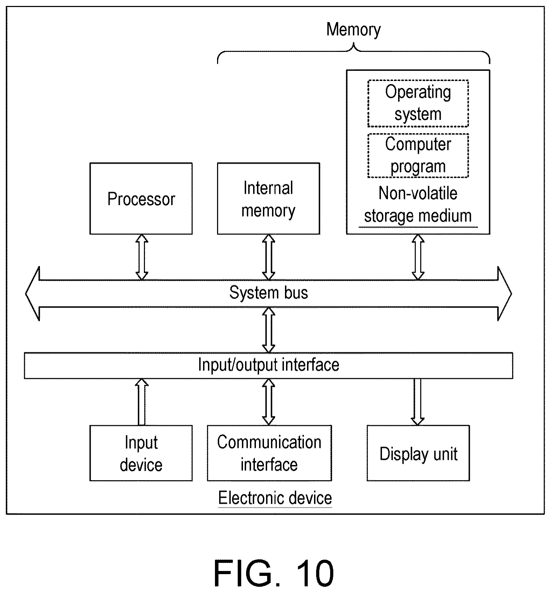

In an embodiment, an electronic device is provided. The electronic device may be a terminal, and a diagram of an internal structure thereof may be as shown in FIG. 10. The electronic device includes a processor, a memory, an input/output interface, a communication interface, a display unit, and an input device. The processor, the memory, and the input/output interface are connected through a system bus. The communication interface, the display unit, and the input device are connected to the system bus through the input/output interface. The processor of the electronic device is configured to provide computing and control capabilities. The memory of the electronic device includes a non-volatile storage medium and an internal memory. The non-volatile storage medium stores an operating system and a computer program. The internal memory provides an environment for the operation of the operating system and computer program of the non-volatile storage medium. The input/output interface of the electronic device is configured to exchange information between the processor and external devices. The communication interface of the electronic device is configured to communicate with external terminals in a wired manner or a wireless manner. The wireless manner may be achieved through WIFI, mobile cellular network, NFC (Near Field Communication) or other technologies. The computer program is executed by the processor to implement the method for analyzing the blood glucose concentration. The display unit of the electronic device is configured to form a visually visible picture, which may be a display screen, a projection device, or a virtual reality imaging device. The display screen may be a liquid crystal display screen or an electronic ink display screen. The input device of the electronic device may be a touch layer covering the display screen, or may be keys, a trackball, or a touchpad provided on a housing of the electronic device, or may be an external keyboard, a touchpad, or a mouse.

Those skilled in the art may understand that the structure shown in FIG. 10 is merely a block diagram of partial structures related to the solution of the present application, and does not constitute a limitation on the electronic device to which the solution of the present application is applied. The specific electronic device may include more or fewer components than those shown in the figure, or combine certain components, or have different component arrangements.

In an embodiment, an electronic device is further provided, including a memory and a processor. The memory stores a computer program. The processor implements the steps of the method embodiments when executing the computer program.

In an embodiment, a computer-readable storage medium is provided, on which a computer program is stored. The computer program implements the steps of the method embodiments when executing the processor.

In an embodiment, a computer program product is provided, including a computer program. The computer program implements the steps of the method embodiments when executed by the processor.

It should be noted that the user information (including but not limited to user device information and user personal information) and data (including but not limited to data used for analysis, stored data, and displayed data) involved in the present application are all information and data authorized by the user or fully authorized by all parties.

Those skilled in the art may understand that implementing all or part of the processes in the methods of the aforementioned embodiments may be accomplished by instructing relevant hardware through the computer program. The computer program may be stored in the non-volatile computer-readable storage medium. When the computer program is executed, the processes of the embodiments of the methods may be included. Any reference to the memory, the database, or other media used in the embodiments provided by the present application may include at least one of the non-volatile memory and the volatile memory. The non-volatile memory may include a read-only memory (ROM), a magnetic tape, a floppy disk, a flash memory, an optical memory, a high-density embedded non-volatile memory, a resistive random access memory (ReRAM), a magnetoresistive random access memory (MRAM), a ferroelectric random access memory (FRAM), a phase change memory (PCM), and a graphene memory. The volatile memory may include a random access memory (RAM) or an external cache memory. As illustration and not limitation, the RAM may be in various forms, such as a static random access memory (SRAM) or a dynamic random access memory (DRAM). The databases involved in the embodiments provided by the present application may include at least one of a relational database and a non-relational database. The non-relational database may include a blockchain-based distributed database, but is not limited thereto. The processors involved in the embodiments provided by the present application may be general-purpose processors, central processing units, graphics processing units, digital signal processors, programmable logic devices, and quantum computing-based data processing logic devices, but are not limited thereto.

The technical features of the aforementioned embodiments may be combined arbitrarily. To make the description concise, not all possible combinations of the technical features in the aforementioned embodiments are described. However, as long as there is no contradiction in the combination of these technical features, these technical features should all be considered within the scope recorded in this specification.

The aforementioned embodiments only express several implementation modes of the present application, and the descriptions thereof are relatively specific and detailed, but should not be understood as limitations on the patent scope of the present application. It should be pointed out that for those skilled in the art, several variations and improvements may be made without departing from the concept of the present application, and these all belong to the protection scope of the present application. Therefore, the protection scope of the present application should be subject to the appended claims.

**Claims**

1. A method for analyzing a blood glucose concentration, **characterized in that**, the method comprises:

   acquiring intensity sets respectively corresponding to a plurality of photoplethysmography pulse wave sets for a target object in a target time period; the intensity sets comprising an average alternating-current intensity and an average direct-current intensity;
   performing curve fitting on the plurality of intensity sets to obtain a fitting coefficient and a fitting bias;
   comparing the fitting bias with a bias range corresponding to the target object, and in a situation when it is determined that the fitting bias is not within the bias range, adjusting the fitting bias to obtain an adjusted fitting bias;
   performing, on a basis of the adjusted fitting bias, the curve fitting on the plurality of intensity sets to obtain an adjusted fitting coefficient, and taking the adjusted fitting coefficient as the blood glucose concentration of the target object in the target time period.

2. The method according to claim 1, **characterized in that**, after performing, on the basis the adjusted fitting bias, the curve fitting on the plurality of intensity sets to obtain the adjusted fitting coefficient, further comprising:

   acquiring a correction parameter value of the target object; the correction parameter value corresponding to a correction parameter, and the correction parameter comprising at least one of physiological characteristic parameters and environmental characteristic parameters;

acquiring a plurality of correction weights corresponding to the target object; the plurality of correction weights comprising a correction weight corresponding to the adjusted fitting coefficient, and correction weights corresponding to each of the plurality of correction parameters;

taking the adjusted fitting coefficient as the blood glucose concentration of the target object in the target time period comprising:

fusing the adjusted fitting coefficient and each of the plurality of correction parameter values with corresponding correction weights to obtain the blood glucose concentration of the target object in the target time period.

3. The method according to claim 1, **characterized in that**, acquiring the plurality of intensity sets corresponding to the plurality of photoplethysmography pulse wave sets for the target object in the target time period comprising:

acquiring the plurality of photoplethysmography pulse wave sets for the target object in the target time period; each photoplethysmography pulse wave set corresponding to one emission intensity, and each photoplethysmography pulse wave set comprising at least two photoplethysmography pulse waves;

for each photoplethysmography pulse wave, performing filtering processing on the photoplethysmography pulse wave to obtain a direct-current signal and an alternating-current signal, on a basis of a signal intensity of the direct-current signal, determining a direct-current intensity corresponding to the photoplethysmography pulse wave, and on a basis of the alternating-current signal, determining an alternating-current intensity corresponding to the photoplethysmography pulse wave;

for each photoplethysmography pulse wave set, averaging direct-current intensities corresponding to the plurality of photoplethysmography pulse waves to obtain an average direct-current intensity, averaging alternating-current intensities corresponding to the plurality of photoplethysmography pulse waves to obtain an average alternating-current intensity, and on a basis of the average direct-current intensity and the average alternating-current intensity, obtaining an intensity set corresponding to the photoplethysmography pulse wave set.

4. The method according to claim 3, **characterized in that**, for each photoplethysmography pulse wave, before performing the filtering processing on the photoplethysmography pulse wave to obtain the direct-current signal and the alternating-current signal, further comprising:

performing a quality evaluation on the photoplethysmography pulse wave to obtain a quality evaluation result of the photoplethysmography pulse wave; the quality evaluation result comprising evaluation values corresponding to each band composing the photoplethysmography pulse wave;

removing bands corresponding to the evaluation values less than an evaluation threshold from the photoplethysmography pulse wave to obtain an adjusted photoplethysmography pulse wave;

for each photoplethysmography pulse wave, performing the filtering processing on the photoplethysmography pulse wave to obtain a direct-current signal and an alternating-current signal comprising:

for each adjusted photoplethysmography pulse wave, performing filtering processing on the adjusted photoplethysmography pulse wave to obtain the direct-current signal and the alternating-current signal.

5. The method according to claim 3, **characterized in that**, on the basis of the alternating-current signal, determining the alternating-current intensity corresponding to the photoplethysmography pulse wave, comprising:

acquiring a plurality of trough coordinates in the alternating-current signal, and performing curve fitting on the plurality of trough coordinates to obtain a fitting signal;

calculating a difference between the alternating-current signal and the fitting signal to obtain a target alternating-current signal;

averaging a plurality of peak intensities in the target alternating-current signal to obtain the alternating-current intensity corresponding to the photoplethysmography pulse wave.

6. The method according to claim 1, **characterized in that**, before comparing the fitting bias with the bias range corresponding to the target object, further comprising:

acquiring a plurality of historical time periods of the target object and a plurality of intensity sets corresponding to each historical time period;

for each historical time period, performing curve fitting on the plurality of intensity sets corresponding to the historical time period to obtain a historical fitting bias; and

obtaining, on a basis of a plurality of historical fitting biases, the bias range corresponding to the target object.

7. The method according to claim 1, **characterized in that**, after comparing the fitting bias with the bias range corresponding to the target object, further comprising:
in a situation when it is determined that the fitting bias is within the bias range, taking the fitting coefficient as the blood glucose concentration of the target object in the target time period.

8. An apparatus for analyzing a blood glucose concentration, **characterized in that**, comprising:

an acquiring module, configured to acquire intensity sets respectively corresponding to a plurality of photoplethysmography pulse wave sets for a target object in a target time period; the plurality of intensity sets comprising an average alternating-current intensity and an average direct-current intensity;
a fitting module, configured to perform curve fitting on the plurality of the intensity sets to obtain a fitting coefficient and a fitting bias;
a comparing module, configured to compare the fitting bias with a bias range corresponding to the target object, and in a situation when it is determined that the fitting bias is not within the bias range, adjust the fitting bias to obtain an adjusted fitting bias;
an adjusting module, configured to perform, on a basis of the adjusted fitting bias, the curve fitting on the plurality of the intensity sets to obtain an adjusted fitting coefficient, and take the adjusted fitting coefficient as the blood glucose concentration of the target object in the target time period.

9. An electronic device, comprising a memory and a processor, the memory storing a computer program, **characterized in that**, the processor implements the steps of the method according to any one of claims 1 to 7 when executing the computer program.

10. A computer-readable storage medium, having a computer program stored thereon, **characterized in that**, the computer program implements the steps of the method according to any one of claims 1 to 7 when executed by a processor.

FIG. 1

```
                                                              ⌒202
┌──────────────────────────────────────────────────────┐
│                                                        │
│      Acquiring intensity sets corresponding to multiple │
│   photoplethysmography pulse wave sets for a target object in a │
│     target time period; the intensity sets including an average │
│     alternating-current intensity and an average direct-current │
│                          intensity                     │
│                                                        │
└──────────────────────────────────────────────────────┘
                             │
                             ▼                ⌒204
┌──────────────────────────────────────────────────────┐
│                                                        │
│    Performing curve fitting on the intensity sets to obtain a fitting │
│                coefficient and a fitting bias          │
│                                                        │
└──────────────────────────────────────────────────────┘
                             │
                             ▼                ⌒206
┌──────────────────────────────────────────────────────┐
│                                                        │
│   Comparing the fitting bias with a bias range corresponding to the │
│   target object, and when it is determined that the fitting bias is not │
│      within the bias range, adjusting the fitting bias to obtain an │
│                      adjusted fitting bias             │
│                                                        │
└──────────────────────────────────────────────────────┘
                             │
                             ▼                ⌒208
┌──────────────────────────────────────────────────────┐
│                                                        │
│   Performing, on the basis of the adjusted fitting bias, curve fitting │
│   on the intensity sets to obtain an adjusted fitting coefficient, and │
│     taking the adjusted fitting coefficient as the blood glucose │
│      concentration of the target object in the target time period │
│                                                        │
└──────────────────────────────────────────────────────┘
```

# FIG. 2

302

Acquiring a correction parameter value of a target object; the correction parameter value corresponding to a correction parameter, and the correction parameter including at least one type of physiological characteristic parameter and environmental characteristic parameter

↓

304

Acquiring multiple correction weights corresponding to the target object; the correction weights including a correction weight corresponding to an adjusted fitting coefficient, and correction weights corresponding to each correction parameter

↓

306

Taking the adjusted fitting coefficient as blood glucose concentration of the target object in a target time period including: fusing the adjusted fitting coefficient and each correction parameter value with the corresponding correction weights to obtain the blood glucose concentration of the target object in the target time period

# FIG. 3

402

Acquiring multiple photoplethysmography pulse wave sets of a target object in a target time period; each photoplethysmography pulse wave set corresponding to one emission intensity, and each photoplethysmography pulse wave set including at least two photoplethysmography pulse waves

↓

404

For each photoplethysmography pulse wave, performing filtering processing on the photoplethysmography pulse wave to obtain a direct-current signal and an alternating-current signal, on the basis of the signal intensity of the direct-current signal, determining a direct-current intensity corresponding to the photoplethysmography pulse wave, and on the basis of the alternating-current signal, determining an alternating-current intensity corresponding to the photoplethysmography pulse wave

↓

406

For each photoplethysmography pulse wave set, averaging direct-current intensities corresponding to multiple photoplethysmography pulse waves to obtain an average direct-current intensity, averaging alternating-current intensities corresponding to the photoplethysmography pulse waves to obtain an average alternating-current intensity, and on the basis of the average direct-current intensity and the average alternating-current intensity obtaining an intensity set corresponding to the photoplethysmography pulse wave set

# FIG. 4

502

Performing a quality evaluation on a photoplethysmography pulse wave to obtain a quality evaluation result of the photoplethysmography pulse wave; the quality evaluation result including evaluation values corresponding to various bands composing the photoplethysmography pulse wave

504

Removing bands corresponding to evaluation values smaller than an evaluation threshold from the photoplethysmography pulse wave to obtain an adjusted photoplethysmography pulse wave

506

For each photoplethysmography pulse wave, performing filtering processing on the photoplethysmography pulse wave to obtain a direct-current signal and an alternating-current signal including: for each adjusted photoplethysmography pulse wave, performing filtering processing on an adjusted photoplethysmography pulse wave to obtain the direct-current signal and the alternating-current signal

# FIG. 5

(a) Good quality of data collection

(b) Poor quality of data collection

Normalization

Time (sec)

Data collection
Quality evaluation

FIG. 6

FIG. 7

FIG. 8

Apparatus for analyzing a blood glucose concentration
~902
Acquiring module

~904
Fitting module

~906
Comparing module

~908
Adjusting module

# FIG. 9

Memory

Operating
system

Computer
program

Non-volatile
storage medium

Processor

Internal
memory

System bus

Input/output interface

Input
device

Communication
interface

Display unit

Electronic device

FIG. 10

## INTERNATIONAL SEARCH REPORT

| International application No. |
|---|
| **PCT/CN2024/075234** |

| A. | CLASSIFICATION OF SUBJECT MATTER |
|---|---|

A61B5/1455(2006.01)i; A61B5/145(2006.01)i; A61B5/00(2006.01)i; G06F17/18(2006.01)i

According to International Patent Classification (IPC) or to both national classification and IPC

| B. | FIELDS SEARCHED |
|---|---|

Minimum documentation searched (classification system followed by classification symbols)

IPC: A61B G06F

Documentation searched other than minimum documentation to the extent that such documents are included in the fields searched

Electronic data base consulted during the international search (name of data base and, where practicable, search terms used)

CNABS; CNTXT; CNKI; VEN; ENTXT; ENTXTC; WPABSC; IEEE: 血糖, 葡萄糖, 光电容积, 交流, 直流, 拟合, 拟合系数, 偏置, 斜率, 截距, 调整, 调节, PPG, AC, DC, blood glucose, alternating current, direct current, fit, match, coefficient, offset, slope, adjust

| C. | DOCUMENTS CONSIDERED TO BE RELEVANT |
|---|---|

| Category* | Citation of document, with indication, where appropriate, of the relevant passages | Relevant to claim No. |
|---|---|---|
| A | CN 115067938 A (NANJING UNIVERSITY OF POSTS AND TELECOMMUNICATIONS) 20 September 2022 (2022-09-20) description, paragraphs [0056]-[0097], and figures 1-4 | 1-10 |
| A | CN 107361776 A (QIANHAI ANYCHECK INFORMATION TECHNOLOGY COMPANY) 21 November 2017 (2017-11-21) entire document | 1-10 |
| A | CN 114943249 A (FUZHOU JIUHOU BIOMEDICAL TECHNOLOGY CO., LTD.) 26 August 2022 (2022-08-26) entire document | 1-10 |
| A | CN 104136918 A (LIFESCAN SCOTLAND LTD.) 05 November 2014 (2014-11-05) entire document | 1-10 |
| A | KR 20210101895 A (PUKYONG NATIONAL UNIVERSITY INDUSTRY-UNIVERSITY COOPERATION FOUNDATION) 19 August 2021 (2021-08-19) entire document | 1-10 |

| ☐ Further documents are listed in the continuation of Box C. | ☑ See patent family annex. |
|---|---|

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "D" | document cited by the applicant in the international application | "X" | document of particular relevance; the claimed invention cannot be considered novel or cannot be considered to involve an inventive step when the document is taken alone |
| "E" | earlier application or patent but published on or after the international filing date | | |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **23 April 2024** | **01 May 2024** |

| Name and mailing address of the ISA/CN | Authorized officer |
|---|---|
| **China National Intellectual Property Administration (ISA/ CN)** **China No. 6, Xitucheng Road, Jimenqiao, Haidian District, Beijing 100088** | |
| | Telephone No. |

Form PCT/ISA/210 (second sheet) (July 2022)

**INTERNATIONAL SEARCH REPORT**
Information on patent family members

| International application No. |
| --- |
| **PCT/CN2024/075234** |

| Patent document cited in search report | | | Publication date (day/month/year) | Patent family member(s) | Publication date (day/month/year) |
| --- | --- | --- | --- | --- | --- |
| CN | 115067938 | A | 20 September 2022 | None | |
| CN | 107361776 | A | 21 November 2017 | None | |
| CN | 114943249 | A | 26 August 2022 | None | |
| CN | 104136918 | A | 05 November 2014 | None | |
| KR | 20210101895 | A | 19 August 2021 | None | |

Form PCT/ISA/210 (patent family annex) (July 2022)

**REFERENCES CITED IN THE DESCRIPTION**

**Patent documents cited in the description**

- CN 202310483892